# EUROPÄISCHE PATENTANMELDUNG

(11) **EP 0 824 014 A1**
(43) Veröffentlichungstag der Anmeldung: **18.02.1998**
(21) Anmeldenummer: 97111664.5
(22) Anmeldetag: 09.07.1997
(51) Int. Cl.: A61F 5/01, A61F 13/06

(54) **Bandage zur Fixierung des Sprunggelenks**

(30) Priorität: 14.08.1996 DE 29614127 U
(71) Anmelder: Kessler, Sigurd, Dr., D-82178 Puchheim (DE)
(72) Erfinder: Kessler, Sigurd, Dr., D-82178 Puchheim (DE)
(74) Vertreter: Finsterwald, Manfred, Dipl.-Ing., Dipl.-Wirtsch.-Ing.

(57) **Zusammenfassung**

Die Erfindung betrifft eine Bandage zur Fixierung des Sprunggelenks mit wenigstens einem fußaußenseitig vorgesehenen, sich zumindest im wesentlichen in Richtung des Außenknöchels erstreckenden Zugband, das mittels einer den Knöchelbereich umschlingenden, am jeweiligen Zugband angreifenden Ringbandage spannbar ist. Bei der erfindungsgemäßen Bandage weist jedes freie Zugbandende einen Befestigungsabschnitt zur Befestigung an einem fußfesten Teil auf.

## Beschreibung

Die vorliegende Erfindung betrifft eine Bandage zur Fixierung des Sprunggelenks mit wenigstens einem fußaußenseitig vorgesehenen, sich zumindest im wesentlichen in Richtung des Außenknöchels erstreckenden Zugband, das mittels einer den Knöchelbereich umschlingenden, am jeweiligen Zugband angreifenden Ringbandage spannbar ist.

Verletzungen von Bändern im Bereich des Sprunggelenks treten in der Praxis sehr häufig auf und sind im Regelfall eine Folge eines Umknickens des Fußes. Dabei kommt es zu Dehnungen, Rissen oder Einrissen von Bändern, die sehr schmerzhaft sind. Besonders häufig betroffen ist dabei das sich in Richtung des Vorderfußes zwischen dem Wadenbein und dem Sprungbein erstreckende Band.

Um eine Heilung der Verletzung zu ermöglichen und den auftretenden Schmerzen entgegenzuwirken, wird das Sprunggelenk durch Anlegen eines Gipses, Anbandagieren einer L-Schiene oder einen sonstigen Stillhalteverband ruhiggestellt. Nachteilig ist dabei, daß durch diese Ruhigstellung der betroffene Patient sein normales Schuhwerk für längere Zeit nicht mehr benutzen kann und dadurch entsprechend behindert ist, und daß überdies im Regelfall das gesamte Sprunggelenk immobilisiert wird, was nach dem Ausheilen der Verletzung aufwendige krankengymnastische Beweglichkeitsübungen erforderlich macht.

Darüber hinaus haben diese starren Verbände den Nachteil, daß sie lediglich zur Behandlung bereits entstandener Verletzungen einsetzbar sind, sie jedoch aufgrund ihrer starren Ausbildung nicht zur Vorbeugung gegen die Entstehung derartiger Verletzungen verwendet werden können.

Aus der DE 4 301 145 A1 ist eine Bandage zur Fixierung des Sprunggelenks bekannt, die eine den Mittelfußbereich umspannende Haltebandage und ein mit dieser Haltebandage fußaußenseitig verbundenes, sich zumindest im wesentlichen in Richtung des Außenknöchels erstreckendes Zugband umfaßt. Dabei ist das Zugband mittels einer den Knöchelbereich umschlingenden, am Zugbandende angreifenden Ringbandage spannbar. Durch diese Bandage wird erreicht, daß der äußere Fußrand in einer leicht angehobenen Stellung fixiert werden kann, wodurch das geschädigte Band entlastet wird, wobei gleichzeitig die Flexibilität des Knöchelgelenkes in den übrigen Richtungen, insbesondere in der wichtigen Vorwärtsrichtung erhalten bleibt.

Problematisch an der Bandage nach der DE 4 301 145 A1 ist jedoch, daß die den Mittelfußbereich umspannende Haltebandage und damit auch das mit der Haltebandage verbundene Zugband nicht in allen Fällen ortsfest am Fuß fixiert werden können. So kann sich die Haltebandage beispielsweise um den Mittelfuß herum verdrehen, wobei gleichzeitig das mit der Haltebandage verbundene Zugbandende verschoben wird. Auf diese Weise kann die durch das gespannte Zugband bewirkte Stabilisierung des Knöchelgelenks zunichte gemacht werden, so daß die Bandage ihre Funktion nicht mehr erfüllt.

Aufgabe der vorliegenden Erfindung ist es, eine Bandage zur Fixierung des Sprunggelenks so auszubilden, daß in allen Fällen eine sichere Stabilisierung des Fußgelenkes erreicht werden kann, so daß bei einer bereits bestehenden Verletzung das geschädigte Band entlastet wird, wobei gleichzeitig die Flexibilität des Knöchelgelenks in den übrigen Richtungen erhalten bleiben soll. Weiterhin soll eine solche Bandage so ausgebildet sein, daß auch eine prophylaktische Anwendung problemlos möglich ist.

Diese Aufgabe wird ausgehend von einer Bandage der eingangs genannten Art dadurch gelöst, daß jedes freie Zugbandende einen Befestigungsabschnitt zur Befestigung an einem fußfesten Teil aufweist.

Durch eine erfindungsgemäß ausgebildete Bandage wird die gestellte Aufgabe vorteilhaft gelöst. Insbesondere kann die Bandage sowohl bei einer bereits bestehenden Verletzung als auch als Prophylaxe verwendet werden, da die Beweglichkeit des Knöchelgelenks ausschließlich in der gewünschten, seitlichen Richtung begrenzt wird. Weiterhin wird durch die Fixierung des oder der Zugbandenden an einem fußfesten Teil, wie es beispielsweise eine Sohle, eine Teilsohle, ein Innenschuh, ein Schuh oder ein Schuhüberzieher darstellt, eine bezüglich des Fußes unverschiebbare Angriffsposition definiert, so daß eine einmal eingestellte Bandage in dieser eingestellten Position sicher fixiert bleibt.

Wesentlich für die Erfindung ist, daß wenigstens ein Zugband vorgesehen ist, das in etwa entsprechend dem beschädigten oder gerissenen bzw. zu schützenden Band verläuft und zwischen zwei durch den Befestigungspunkt an dem fußfesten Teil und die Ringbandage geschaffenen Festpunkten gespannt werden kann. Auf diese Weise ist es möglich, den äußeren Fußrand in insbesondere leicht angehobener Stellung zu fixieren und damit das geschädigte bzw. zu schützende Band zu entlasten. Da sämtliche Bestandteile der Bandage flach ausgebildet sind, ist die Bandage nicht störend, so daß eine hohe Akzeptanz insbesondere auch als prophylaktische Maßnahme erreicht werden kann.

Insbesondere zur Prophylaxe kann beispielsweise ein Sportschuh mit einer erfindungsgemäß ausgebildeten Bandage kombiniert werden. Auf diese Weise kann anstelle eines üblichen knöchelhohen Schuhes ein niedrig geschnittener Sportschuh mit erfindungsgemäßer Bandage verwendet werden, der ein Umknicken des Fußes ebensogut oder besser als der knöchelhohe Sportschuh verhindert.

Dabei ist vorteilhaft, daß zum einen die Beweglichkeit des Knöchelgelenks bei einem Sportschuh mit niedrigem Schaft größer ist als die Beweglichkeit bei einem knöchelhohen Sportschuh, so daß nach Anlegen der Bandage bei einem niedrig geschnittenen Schuh - wodurch die Beweglichkeit lediglich in seitlicher Richtung eingeschränkt wird - eine insgesamt bessere Beweglichkeit des Knöchelgelenkes erreicht wird. Darüber hinaus kann durch die Verwendung eines niedrig geschnittenen Schuhs das durch die Schuhe bedingte Gewicht verringert werden, was insbesondere bei Sportarten wie Basketball oder Volleyball vorteilhaft ist.

In einer besonders vorteilhaften Ausführungsform besitzt das fußfeste Teil der Bandage eine sohlenförmige oder teilsohlenförmige, durch den Vorderfuß belastbare Kuppelfläche, an welcher der Befestigungsabschnitt jedes Zugbandes derart befestigbar ist, daß die Länge, die Spannung und/oder der Kraftanlenkpunkt des jeweiligen Zugbandes variabel einstellbar ist. In dieser Ausführungsform kann das fußfeste Teil bzw. die erfindungsgemäße Bandage besonders platzsparend ausgebildet sein, so daß bei angelegter Bandage bereits vorhandene Schuhe des Benutzers verwendet werden können.

Indem die Kuppelfläche zwischen dem fußfesten Teil und jedem Zugband durch den Vorderfuß belastbar ist, kann das Körpergewicht auf die Kuppelfläche übertragen werden. Durch die Belastung mit dem Körpergewicht erweist sich die Verbindung an der Kuppelfläche als besonders stabil gegen unbeabsichtigtem Lösen, Nachgeben oder Verrutschen, was insbesondere bei lösbaren Verbindungen von großem Vorteil ist. Die variable Einstellung von Länge, Spannung, Kraftanlenkpunkt oder Zugrichtung der Zugbänder ermöglicht eine den Bedürfnissen des Anwendungsfalles, wie beispielsweise der Art der Verletzung, optimale Anpassung der Bandage. Insbesondere ist es möglich, daß der Benutzer, beispielsweise während des Heilungsprozesses, die Zugkräfte bzw. die Bandlängen nach eigenem Schmerzempfinden einstellt.

Wenn das fußfeste Teil im wesentlichen flach ausgebildet ist, aus halbsteifem Material besteht und/oder an eine Fußsohlenform anatomisch anpaßbar ist, wird ein Nachgeben oder Verrutschen des fußfesten Teils bei ruckartigem Anspannen des oder der Zugbänder besonders wirkungsvoll verhindert. Ferner kann das fußfeste Teil in vorteilhafter Weise als Einlagesohle ausgebildet sein und, gegebenenfalls nach Entfernung der bisher verwendeten Schuheinlage, in einen bereits vorhandenen Schuh des Benutzers eingelegt werden. Das fußfeste Teil kann sich insbesondere über den gesamten Bereich der Fußsohle oder nur über einen Teilbereich erstrecken.

Es ist bevorzugt, wenn die Kuppelfläche des fußfesten Teils zur Befestigung des jeweiligen Befestigungsabschnittes jedes Zugbandes mit einem einen flächigen Bereich einnehmenden Klettverschluß- oder Haftklebeorgan ausgebildet ist. Dementsprechend kann der jeweilige Befestigungsabschnitt jedes Zugbandes zur Verbindung mit dem fußfesten Teil als Klettverschluß- oder Haftklebeelement ausgebildet sein. In diesem Fall kann die Verbindung des oder der Zugbänder mit dem fußfesten Teil lösbar und gleichzeitig besonders platzsparend ausgebildet sein, so daß für die erfindungsgemäße Bandage ein hoher Tragekomfort erreicht wird. Wenn das Verbindungsorgan an der Unterseite des fußfesten Teils angebracht ist, ist es für den Benutzer der Bandage kaum spürbar.

In einer weiteren vorteilhaften Ausführungsform ist jedes der Ringbandage abgewandte Zugbandende mit dem fußfesten Teil fest verbunden, insbesondere vernäht. Die Einstellung der Spannung am jeweiligen Zugband erfolgt in diesem Fall durch eine Längenverstellung des Zugbandes oder an dem der Ringbandage zugewandten Zugbandende. Bevorzugt ist das jeweilige Zugband mit der Ringbandage mittels einer Verbindungsschlaufe verbunden, die insbesondere über eine Klettverschlußverbindung verstellbar ist.

Zur unterstützenden Fixierung des fußfesten Teils am Fuß kann dieses ein oder mehrere Fixierbänder aufweisen, die an dem fußfesten Teil befestigt sind und entlang der Fußseiten und der Fußoberseite, insbesondere über Kreuz, verlaufen. Der Fuß kann somit im fußfesten Teil aufgenommen werden. Es ist von Vorteil, wenn die Fixierbänder dabei insbesondere über eine Klettverschlußverbindung längenverstellbar sind. Der jeweilige Befestigungsabschnitt jedes freien Zugbandendes kann sowohl zur Befestigung direkt an dem fußfesten Teil als auch zur Befestigung an einem der Fixierbänder bzw. an dem Fixierband ausgebildet sein.

In einer weiteren vorteilhaften Ausführungsform ist das fußfeste Teil als Innenschuh ausgebildet, in welchem der Fuß aufnehmbar ist und der die sohlenförmige oder teilsohlenförmige Kuppelfläche am Fuß fixiert. Der Innenschuh kann dabei als Überziehstrumpf aufgebaut sein, an oder in dem eine Einlagesohle befestigt ist. Somit ist auch diese Ausführungsform vorteilhaft mit vorhandenem Schuhwerk des Benutzers verwendbar.

In einer zu den vorgenannten Ausführungsformen alternativen Ausgestaltung ist das fußfeste Teil als Schuh oder Schuhüberzieher ausgebildet und weist wenigstens ein fußaußenseitig, insbesondere im Mittelfußbereich vorgesehenes Befestigungselement zur Befestigung der jeweiligen Zugbänder auf. Dabei entspricht die Anzahl der Befestigungselemente der Anzahl der freien Zugbandenden. Bei dieser Ausführungsform besteht für den Benutzer der erfindungsgemäß ausgebildeten Bandage keinerlei Beeinträchtigung des Tragegefühls.

Ein bereits vorhandener Schuh des Benutzers, beispielsweise ein Sportschuh, ein Halbschuh oder eine Sandale, kann mit sehr einfachen Mitteln zur Verwendung mit der Bandage umgerüstet werden, indem beispielsweise fußaußenseitig an diesem Schuh wenigstens eine Öse befestigt wird, an der das jeweilige Zugbandende befestigbar ist. Wenn das fußfeste Teil als Schuhüberzieher ausgebildet ist, kann vorhandenes Schuhwerk ebenfalls benutzt werden. Schließlich kann auch ein speziell für orthopädische Zwecke ausgebildeter medizinischer Schuh mit der erfindungsgemäßen Bandage versehen sein.

Bevorzugt ist das Befestigungselement an der Außenseite des Schuhs bzw. des Schuhüberziehers ausgebildet. Dadurch ist zum einen eine besonders einfache Befestigung jedes freien Zugbandendes und ein einfaches Anlegen der Bandage, z. B. bei bereits angezogenem Schuh, möglich; zum anderen werden störende Druckstellen unmittelbar am Fuß vermieden. Grundsätzlich kann das Befestigungselement auch an der Innenseite des Schuhs bzw. des Schuhüberziehers ausgebildet sein. t

Der jeweilige Befestigungsabschnitt zur Verbindung des Zugbandes mit dem Befestigungselement am Schuh bzw. Schuhüberzieher kann vorteilhaft ein Klettverschluß-, ein Haftklebe-, ein Knöpf-, ein Einhäng- oder ein Schnürverbindungselement umfassen. So kann beispielsweise an dem Schuh bzw. dem Schuhüberzieher eine Öse angebracht sein, durch die das Zugband hindurchgezogen wird, wonach anschließend das freie Zugbandende um ca. 180° zurückgeschlagen und mit dem in Richtung des Knöchels weisenden Abschnitt des Zugbandes verbunden wird. Diese Verbindung kann durch am Zugband vorgesehene Klettverschlußelemente oder sonstige übliche Verbindungselemente erfolgen.

Um ein Spannen des Zugbandes zu ermöglichen, kann jedes Zugband in seiner Länge verstellbar ausgebildet sein. Insbesondere kann sich das am knöchelseitigen Abschnitt des Zugbandendes vorgesehene Klettverschlußelement über einen größeren Bereich erstrecken, so daß das durch die Öse hindurch umgeschlagene freie Zugbandende in unterschiedlichen Positionen fixiert werden kann.

Bei einer Knöpfverbindung können beispielsweise mehrere versetzt angeordnete Knopflöcher bzw. Knöpfe vorgesehen sein, so daß auf diese Weise eine Verstellbarkeit und damit verbunden eine Spannbarkeit des Zugbandes erreichbar ist. Auf ähnliche Weise kann bei einer Haftklebe- oder einer Einhäng-Verbindung eine Spannbarkeit des Zugbandes erreicht werden.

Bei den verschiedenen möglichen Ausführungsformen des fußfesten Teils kann die Ringbandage mit einem einzigen freien Zugbandende unter Ausbildung einer Y-Bandverzweigung oder mit zwei freien Zugbandenden unter Ausbildung einer X-Bandverzweigung verbunden sein, um eine wirkungsvolle und ortsfeste Kraftübertragung zu ermöglichen. Es ist möglich, die Ringbandage mit dem oder den Zugbändern über ein Ringelement zu verbinden. Jedes Zugband kann mit dem Ringelement fest oder in einer insbesondere über eine Klettverschlußverbindung verstellbaren Verbindungsschlaufe verbunden sein.

Nach einer weiteren vorteilhaften Ausführungsform der Erfindung ist fußinnenseitig ein weiteres Zugband vorgesehen, das mittels der Ringbandage spannbar ist und dessen freies Ende einen weiteren Befestigungsabschnitt zur Befestigung an einem fußfesten Teil, insbesondere an einem Sohlenabschnitt, einem Schuh oder einem Schuhüberzieher, aufweist. Auf diese Weise wird erreicht, daß sowohl eine Stabilisierung des Knöchelgelenkes nach außen als auch nach innen durch die erfindungsgemäß ausgebildete Bandage erzielbar ist, wobei gleichzeitig weiterhin die Beweglichkeit des Knöchelgelenks in den übrigen Richtungen, insbesondere in der wichtigen Vorwärtsrichtung weitgehend ungehindert erhalten bleibt.

Ein erfindungsgemäß ausgebildeter Schuh oder Schuhüberzieher ist durch ein fußaußenseitig, insbesondere im Mittelfußbereich vorgesehenes Befestigungselement zur Befestigung einer Fußbandage, insbesondere des freien Zugbandendes einer erfindungsgemäß ausgebildeten Bandage gekennzeichnet. Insbesondere durch die nachträgliche Anbringbarkeit des Befestigungselements an dem Schuh oder dem Schuhüberzieher können vorhandene Schuhe verwendet werden, so daß zum einen der Tragekomfort und zum anderen die Akzeptanz der Bandage erhöht wird.

Weitere vorteilhafte Ausführungsformen sind in den Unteransprüchen angegeben.

Im weiteren wird die Erfindung anhand von Ausführungsbeispielen unter Bezugnahme auf die Figuren näher erläutert; in diesen zeigen:
- Figur 1a: eine schematische Seitenansicht einer an einem Fuß angelegten erfindungsgemäßen Bandage,
- Figur 1b:: die Unterseite des fußfesten Teils der Bandage gemäß Figur 1a, und
- Figur 2:: eine schematische Darstellung einer weiteren erfindungsgemäßen Bandage im angelegten Zustand.

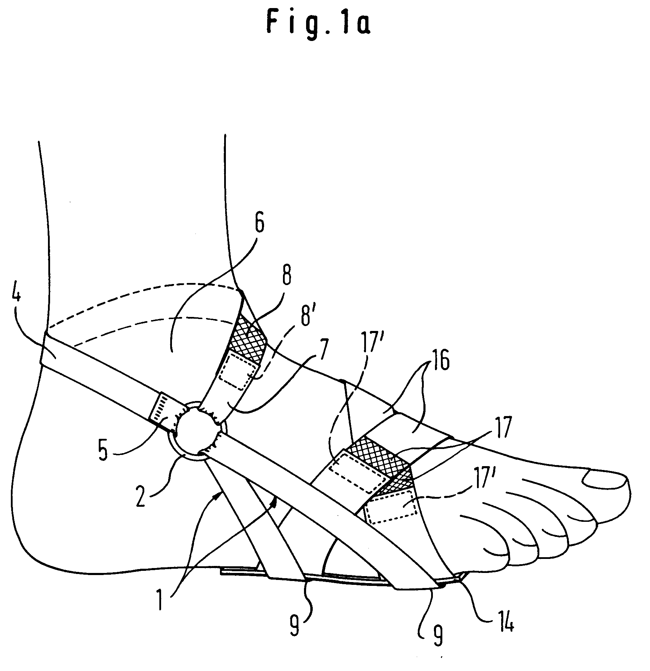

Figur 1a zeigt in schematischer Ansicht der Fußaußenseite eine erfindungsgemäß ausgebildete Bandage im angelegten Zustand. Die Bandage weist ein Zugband 1 auf, das durch einen im Knöchelbereich 6 befindlichen Ring 2 frei beweglich geschleift ist. In Folge der Umlenkung am Ring 2 weist ein Teilstück des Zugbandes 1 in Richtung des fußaußenseitigen Rands des Vorderfußes, und das andere Teilstück des Zugbandes 1 weist in Richtung des fußaußenseitigen Rands des Mittelfußes.

An dem Ring 2 ist eine den Knöchelbereich 6 umschlingende Ringbandage 4 befestigt. Hierfür ist ein Ende 5 der Ringbandage 4 mit dem Ring 2 vernäht und somit fest verbunden. Das andere Ende 7 der Ringbandage 4 ist durch den Ring 2 geschleift und um 180° umgeschlagen. Das Ringbandagenende 7 weist an seiner in Figur 1a nicht sichtbaren Seite im gestrichelt umrandeten Bereich ein Klettverschlußorgan 8' auf, das mit einem sich entlang der Ringbandage 4 erstreckendem Klettverschlußorgan 8 lösbar haftend verbunden ist. Durch die zusammenwirkenden Klettverschlußorgane 8, 8' läßt sich die Ringbandage 4 um den Knöchelbereich 6 anlegen, wobei sich verschiedene Längen der Ringbandage 4 bzw. verschiedene Positionen des Rings 2 einstellen lassen.

Im Bereich des Vorderfußes und einem Teil des Mittelfußes liegt ein fußfestes Teil 14 an der Unterseite des Fußes an. Das fußfeste Teil 14 ist flach und aus halbsteifen, verformbaren Material ausgebildet. Sein Umriß entspricht einem Abschnitt des Umrisses der Fußunterseite.

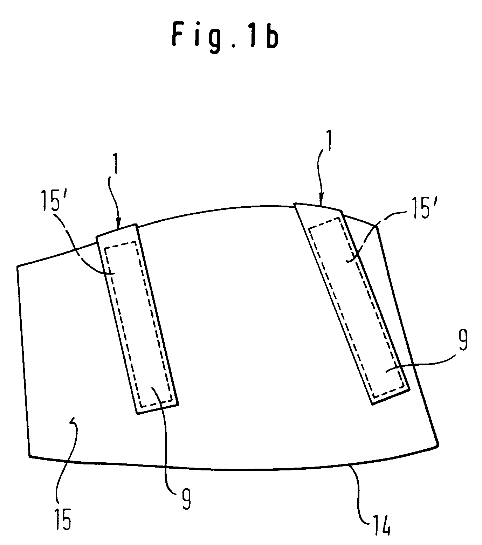

In Figur 1b ist die Unterseite des fußfesten Teils 14 dargestellt, wobei im oberen Bereich der Figur 1b der Außenlängsrand und im unteren Bereich der Innenlängsrand des fußfesten Teils 14 gezeigt ist. Die gesamte in Figur 1b dargestellte Unterseite des fußfesten Teils 14 ist als Kuppelfläche 15, beispielsweise als Klettverschlußorgan, ausgestaltet.

Die dem Ring 2 abgewandten Teilstücke des Zugbandes 1 sind am fußaußenseitigen Längsrand des fußfesten Teils 14 umgelenkt, so daß die beiden Zugbandenden 9 entlang der Unterseite des fußfesten Teils 14 verlaufen. Die jeweils der Fußunterseite zugewandte Seite jedes Zugbandendes 9 weist einen Befestigungsabschnitt auf, der als Klettverschlußorgan (15') ausgebildet ist, welcher mit der Kuppelfläche 15 lösbar haftend verbunden ist. Die beiden Klettverschlußorgane (15') sind in Figur 1b nicht sichtbar; sie sind jedoch durch gestrichelte Linien angedeutet.

An den beiden Längsrändern der Oberseite des fußfesten Teils 14 sind jeweils die Enden zweier Fixierbänder 16 befestigt, beispielsweise angenäht. Jedes Fixierband 16 verläuft entlang der Fußaußenseite über die Oberseite des Vorderfußes zur Fußinnenseite. Dabei verlaufen beide Fixierbänder 16 über Kreuz. Die Fixierbänder 16 sind jeweils aus zwei Teilstücken aufgebaut, an deren jeweiligem, dem anderen Teilstück zugewandten Ende ein Klettverschlußorgan 17 bzw. 17' angebracht ist. Die Klettverschlußorgane 17, 17' wirken zusammen, so daß sich die beiden Teilstücke jedes Fixierbandes 16 zu unterschiedlichen Gesamtlängen lösbar haftend verbinden lassen.

Die in den Figuren 1a und 1b dargestellte erfindungsgemäß ausgebildete Bandage läßt sich an den Fuß anlegen, indem zunächst die beiden Klettverschlußorgane 8 und 8' der Ringbandage 4 geöffnet werden, die Ringbandage 4 um den Knöchelbereich 6 des Fußes angelegt wird und die Klettverschlußorgane 8 und 8' derart miteinander verbunden werden, daß die Ringbandage 4 dem Knöchelbereich 6 eng anliegt. Das Zugband 1 kann dabei bereits durch den Ring 2 geführt sein, ist jedoch noch nicht an dem fußfesten Teil 14 befestigt.

Anschließend wird der Vorderfuß bei geöffneten Fixierbändern 16 auf die Oberseite des fußfesten Teils 14 gesetzt, und die jeweiligen Teilstücke jedes Fixierbandes 16 werden mittels der Klettverschlußorgane 17 und 17' miteinander verbunden, so daß die Fixierbänder 16 den Vorderfuß straff umspannen.

Schließlich wird das Zugband 1 zwischen der Ringbandage 4 und dem fußfesten Teil 14 angespannt, indem seine beiden dem Ring 2 abgewandten Teilstücke über den äußeren Fußrand umgelenkt werden und die beiden Enden 9 des Fußbandes 1 mit der Kuppelfläche 15 verbunden werden. Die Bandage ist somit entsprechend der in Figur 1a gezeigten Darstellung am Fuß angelegt und gespannt. Über den dergestalt bandagierten Fuß kann eine Socke und/oder ein normaler Schuh des Benutzers angelegt werden.

Beim Anlegen der Zugbandenden 9 an das fußfeste Teil 14 ist die zwischen der Ringbandage 4 und dem fußfesten Teil 14 fußaußenseitig herrschende Spannung frei wählbar. Auch der jeweilige Kraftumlenkungspunkt jedes Zugbandendes 9 an der Fußaußenseite ist individuell einstellbar. Somit läßt sich die erfindungsgemäße Bandage den jeweiligen Anwendungsbedingungen, wie beispielsweise einer prophylaktischen Benutzung oder der Lage von verletzten Bändern, individuell anpassen.

Das fußfeste Teil 14 ist bei Benutzung der erfindungsgemäßen Bandage an der Unterseite des Fußes fest fixiert, da es zwischen der Fußunterseite und einem angelegten Schuh eingespannt ist. Die über die Fußunterseite übertragene Belastung durch das Körpergewicht verhindert ein Verrutschen des fußfesten Teils 14, insbesondere genau dann, wenn eine bezüglich einer möglichen oder bereits erfolgten Verletzung besonders starke Belastung des Fußes erfolgt. Ferner ist die Form des fußfesten Teils 14 der Anatomie der Fußunterseite angepaßt, so daß sich eine weitere Stabilisierung ihrer Lage relativ zum Fuß ergibt.

Die Fixierbänder 16 verhindern ein Verrutschen des fußfesten Teils insbesondere in Richtung der Fußferse. Eine Ausgestaltung des fußfesten Teils 14 mit den Fixierbändern 16 ist daher bevorzugt, jedoch nicht zwingend notwendig. Ein Verrutschen des fußfesten Teils 14 in Längsrichtung des Fußes kann beispielsweise auch dadurch verhindert werden, däß das zwischen der Fußunterseite und einem angelegten Schuh eingespannte fußfeste Teil als Einlagesohle ausgebildet ist, deren Umriß den gesamten Umriß der Fußunterseite entspricht. Weiterhin müssen die Fixierbänder 16 nicht durch Klettverschlußorgane 17, 17' längenverstellbar sein, sondern können auch als durchgehende Bänder, beispielsweise Gummibänder, ausgestaltet sein. In diesem Fall wird das fußfeste Teil 14 dem Fuß angelegt, indem der Vorderfuß in die durch die Bänder und das fußfeste Teil 14 gebildete Öffnung eingeführt wird, bis die Bänder an der Fußoberseite und das fußfeste Teil 14 an der Fußunterseite fest anliegen.

Indem das Zugband 1 frei beweglich durch den Ring 2 geschleift ist, läßt sich eine Gesamtspannung einstellen, die zwischen der Ringbandage 4 und dem fußfesten Teil 14 herrscht. Hierdurch läßt sich bezüglich einer Auf- und Abwärtsbewegung der Fußspitze eine hohe Bewegungsfreiheit erreichen. Es ist jedoch auch möglich, das Zugband 1 mit dem Ring 2 fest zu verbinden, beispielsweise zu vernähen, so daß die Spannung zwischen der Ringbandage 4 und jedem der beiden Zugbandenden 9 bzw. deren Anlenkungspunkten am fußfesten Teil 14 einzeln einstellbar ist.

Die verschiedenen Bestandteile der Bandage, insbesondere das fußfeste Teil 14, können an der dem Fuß zugewandten Seite jeweils aus einem haftenden Material bestehen, so daß die Bandage noch stabiler gegenüber einem Verrutschen bei hohen Zugspannungen ist. In diesem Fall ist es sinnvoll, eine Socke erst bei bereits eingelegter Bandage dem Fuß überzuziehen.

Weiterhin kann zur Erhöhung des Tragekomforts der Bandage der Ring 2 flach ausgeführt sein. Eine gepolsterte Abdeckung, die beispielsweise an der Ringbandage 4 befestigt ist, kann zwischen dem Ring 2 und dem Knöchelbereich 6 des Fußes angeordnet sein.

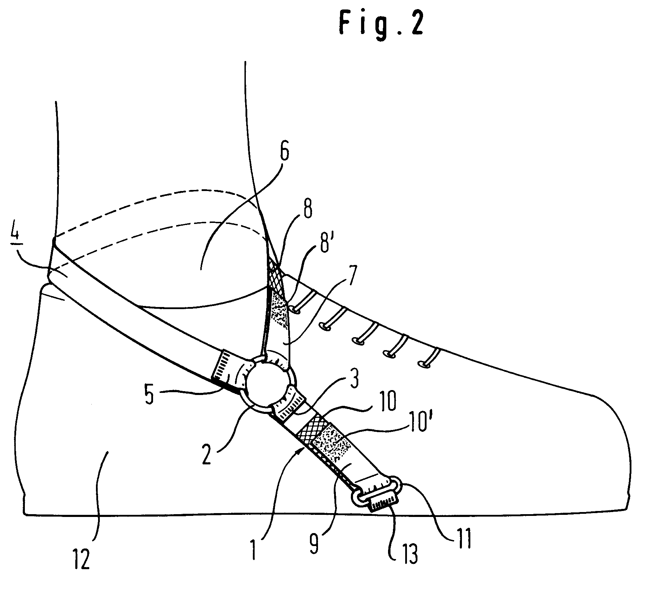

In Figur 2 ist eine weitere erfindungsgemäß ausgebildete Bandage schematisch dargestellt. Diese weist ein Zugband 1 auf, dessen knöchelseitiges Ende an einem Ring 2 befestigt ist. Zur Befestigung an dem Ring 2 bildet das knöchelseitige Ende des Zugbands 1 eine Schlaufe, die in den Ring 2 eingehängt und über die Breite des Zugbandes 1 durch Nahtstellen 3 gesichert ist.

In gleicher Weise ist eine Ringbandage 4 mit seinem einen Ende 5 an dem Ring 2 befestigt.

Die Ringbandage 4 umschlingt den Knöchelbereich 6 und ist mit seinem anderen Ende 7 ebenfalls an dem Ring 2 befestigt. Dazu ist an einer Seite der Ringbandage 4 im Bereich vor dessen freien Ende 7 ein Klettverschlußorgan 8 vorgesehen, das mit einem im Bereich des freien Endes 7 vorgesehenen weiteren Klettverschlußorgan 8' haftend verbunden ist, nachdem das freie Ende 7 durch den Ring 2 hindurchgeführt und ca. um 180° umgeschlagen ist. Zur Verdeutlichung ist in der Figur 2 die Lage des Klettverschlußorgans 8' auf der Rückseite des freien Endes 7 der Ringbandage 4 durch eine graue Fläche dargestellt.

Da sich das Klettverschlußorgan 8 bzw. das mit diesem zusammenwirkende Klettverschlußorgan 8' am freien Ende 7 der Ringbandage über einen größeren Bereich der Ringbandage 4 erstreckt, ist je nach der Länge des umgeschlagenen Endes der Ringbandage 4 eine variable Einstellung der Länge der Ringbandage 4 und damit verbunden eine Positionierung des Rings 2 sowie ein Spannen der erfindungsgemäßen Bandage möglich.

Grundsätzlich kann auch das Ende 5 der Ringbandage 4 ähnlich dem freien Ende 7 verstellbar ausgebildet sein oder es kann auch nur das Ende 5 verstellbar ausgebildet sein, während das freie Ende 7 der Ringbandage 4 fest mit dem Ring 2 verbunden ist, wie es im Ausführungsbeispiel am Ende 5 der Ringbandage 4 dargestellt ist. Während bei einer beidseitigen variablen Befestigung das Spannen der Ringbandage 4 einfacher möglich ist, hat die einseitig variable Befestigung den Vorteil, daß die erfindungsgemäße Bandage insgesamt aus fest zusammenhängenden Elementen besteht, die nicht auseinandernehmbar sind, so daß die Gefahr des Verlierens eines Elementes nicht gegeben ist.

Das fußsohlenseitig gelegene freie Ende 9 des Zugbandes 1 ist ähnlich dem freien Ende 7 der Ringbandage 4 zu einer Schlaufe umgeschlagen, die durch ein im Bereich vor dem freien Ende vorgesehenes Klettverschlußorgan 10 sowie ein am freien Ende 9 des Zugbandes 1 vorgesehenes mit dem Klettverschlußorgan 10 zusammenwirkendes Klettverschlußorgan 10' zusammengehalten wird.

Die von dem freien Ende 9 des Zugbandes 1 gebildete Schlaufe ist in einer Ringöse 11 eingehängt, die fußaußenseitig im Bereich des Mittelfußes nahe der Sohle eines Schuhes 12 über eine Lasche 13 mit dem Schuh 12 verbunden ist. Je nachdem, wie weit das freie Ende 9 des Zugbandes 1 durch die Ringöse 11 hindurchgezogen und über die beiden Klettverschlußorgane 10, 10' verbunden wird, wird eine mehr oder weniger große Spannung des Zugbandes 1 erreicht. Durch diese Spannung kann eine mehr oder weniger große Entlastung der seitlichen Knöchelgelenksbänder, insbesondere des sich zwischen Wadenbein und Sprungbein erstreckenden Bandes erreicht werden.

Durch die erfindungsgemäße Bandage wird somit erreicht, daß die jeweiligen Bänder bzw. das jeweilige Band entlastet wird und demgemäß praktisch keine Zugkräfte auf dieses Band einwirken können, da der äußere Fußrand zumindest in leicht angehobener Stellung fixiert wird. Ein Abwinkeln des Unterschenkels nach vorne sowie ein Abwinkeln des Knöchelgelenks nach vorne oder hinten wird dabei nicht behindert, so daß sowohl das Gehen als auch eine weitgehende Beweglichkeit des Knöchelgelenkes bei angelegter Bandage erhalten bleibt.

Durch die Anlenkung des freien Zugbandendes 9 unmittelbar an einem Befestigungselement am Schuh 12 wird eine bezüglich des Fußes ortsfeste Festlegung des unteren Angriffspunktes der Bandage erreicht, so daß eine einmal eingestellte erfindungsgemäß ausgebildete Bandage während des Tragens seine eingestellte Spannung nicht verändert. Insbesondere ist gewährleistet, daß auch bei einer hohen Belastung der Bandage, wie sie beispielsweise bei einem plötzlichen, seitlichen Umknicken des Fußes auftritt, keine Verschiebung der Angriffspunkte der Bandage auftreten kann, so daß die Bandage sicher in ihrem eingestellten Zustand verbleibt.

Ist neben der fußaußenseitigen Fixierung auch eine fußinnenseitige Fixierung zur Sicherung der fußinnenseitig gelegenen Bänder gewünscht, so kann auch an der fußinnenseitig gelegenen Seite des Schuhs ein entsprechendes Befestigungselement, beispielsweise in Form einer Ringöse vorgesehen sein. In diese Ringöse kann dann entsprechend dem dargestellten Ausführungsbeispiel ein weiteres Zugband befestigt sein, das mit der Ringbandage 4 über ein weiteres fußinnenseitig gelegenes Verbindungselement, beispielsweise in Form eines weiteren Ringes verbunden ist. Die Beweglichkeit des Knöchelgelenks in den übrigen Richtungen, insbesondere nach vorne und nach hinten bleibt auch bei diesem zweiseitigen Ausführungsbeispiel erhalten.

Anstelle der Befestigung direkt an einem Schuh oder einem Schuhüberzieher kann das freie Zugbandende auch an einer Haltebandage befestigt sein, die den Vorder- oder Mittelfuß unmittelbar umschließt. Das Material dieser Haltebandage ist vorteilhaft so gewählt, daß eine rutschfeste Verbindung zwischen der Haltebandage und der Haut des Fußes entsteht. Durch Wahl eines geeigneten Materials, beispielsweise einer Art Kunststoffolie, kann ein Hafteffekt erzielt werden, der eine eindeutige Festlegung des Anlenkpunktes des Zugbandes gewährleistet. Bevorzugt ist das verwendete Material so ausgebildet, daß es sich an den Fuß anschmiegt, um dadurch eine ausreichende Rutschfestigkeit zu erzielen.

Die Breite der Haltebandage ist so zu wählen, daß zum einen die benötigte Fläche zwischen Haltebandage und Fußoberfläche vorhanden ist, die einen ausreichenden Hafteffekt gewährleistet, wobei durch eine genügende Breite auch ein Einschneiden des Haltebandes in den Fuß vermieden wird. Gleichzeitig darf das Halteband jedoch nicht so breit gewählt werden, daß eine Einschränkung im Tragekomfort bzw. der Beweglichkeit des Mittel- bzw. Vorderfußes entsteht. Vorteilhafte Breiten liegen beispielsweise im Bereich zwischen einem und acht Zentimeter.

### Bezugszeichenliste

- 1: Zugband
- 2: Ring
- 3: Nahtstellen
- 4: Ringbandage
- 5: Ringbandagenende
- 6: Knöchelbereich
- 7: Ringbandagenende
- 8: Klettverschlußorgan
- 8': Klettverschlußorgan
- 9: Zugbandende
- 10: Klettverschlußorgan
- 10': Klettverschlußorgan
- 11: Ringöse
- 12: Schuh
- 13: Lasche
- 14: fußfestes Teil
- 15: Kuppelfläche
- 15': Klettverschlußorgan
- 16: Fixierband
- 17: Klettverschlußorgan
- 17': Klettverschlußorgan

## Patentansprüche

1. Bandage zur Fixierung des Sprunggelenks mit wenigstens einem fußaußenseitig vorgesehenen, sich zumindest im wesentlichen in Richtung des Außenknöchels erstreckenden Zugband (1), das mittels einer den Knöchelbereich (6) umschlingenden, am jeweiligen Zugband (1) angreifenden Ringbandage (4) spannbar ist,
**dadurch gekennzeichnet,**
daß jedes freie Zugbandende (9) einen Befestigungsabschnitt zur Befestigung an einem fußfesten Teil (12, 14) aufweist.

2. Bandage nach Anspruch 1,
**dadurch gekennzeichnet,**
daß das fußfeste Teil (14) eine sohlenförmige oder teilsohlenförmige, durch den Vorderfuß belastbare Kuppelfläche (15) aufweist, an welcher der Befestigungsabschnitt (15') jedes Zugbandes (1) derart befestigbar ist, daß die Länge, die Spannung und/oder der Kraftanlenkpunkt des jeweiligen Zugbandes (1) variabel einstellbar ist.

3. Bandage nach Anspruch 2 oder 3,
**dadurch gekennzeichnet,**
daß das fußfeste Teil (14) im wesentlichen flach ausgebildet ist, aus halbsteifem Material besteht und/oder an eine Fußsohlenform anpaßbar ist.

4. Bandage nach einem der vorhergehenden Ansprüche,
**dadurch gekennzeichnet,**
daß die Kuppelfläche (15) des fußfesten Teils (14) zur Befestigung des jeweiligen Befestigungsabschnittes (15') jedes Zugbandes (1) mit einem Klettverschluß- oder Haftklebeorgan ausgebildet ist, das einen flächigen Bereich, insbesondere an der Unterseite des fußfesten Teils (14), einnimmt.

5. Bandage nach einem der vorhergehenden Ansprüche,
**dadurch gekennzeichnet,**
daß der jeweilige Befestigungsabschnitt jedes Zugbandes (1) zur Verbindung mit dem fußfesten Teil (14) ein Klettverschlußelement (15') oder ein Haftklebeelement umfaßt.

6. Bandage nach einem der vorhergehenden Ansprüche,
**dadurch gekennzeichnet,**
daß das fußfeste Teil (14) am Fuß fixierbar ist durch ein oder mehrere Fixierbänder (16), die an dem fußfesten Teil (14) befestigt sind, entlang der Fußseiten und der Fußoberseite verlaufen und längenverstellbar sind, und die insbesondere über eine Klettverschlußverbindung (17, 17') verstellbar sind und/oder über Kreuz verlaufen.

7. Bandage nach einem der Ansprüche 1 bis 5,
**dadurch gekennzeichnet,**
daß das fußfeste Teil (14) als Innenschuh ausgebildet ist, in dem der Fuß aufnehmbar ist und der die sohlenförmige oder teilsohlenförmige Kuppelfläche (15) am Fuß fixiert.

8. Bandage nach Anspruch 1,
**dadurch gekennzeichnet,**
daß das fußfeste Teil als Schuh (12) oder Schuhüberzieher ausgebildet ist und wenigstens ein fußaußenseitig, insbesondere im Mittelfußbereich vorgesehenes Befestigungselement (11) zur Befestigung der jeweiligen Zugbänder (1) aufweist, wobei die Anzahl der Befestigungselemente (11) der Anzahl der freien Zugbandenden (9) entspricht.

9. Bandage nach Anspruch 8,
**dadurch gekennzeichnet,**
daß jedes Befestigungselement (11) an der Außenseite oder der Innenseite des Schuhs (12) bzw. des Schuhüberziehers und/oder im Bereich nahe der Unterseite des Schuhs (12) bzw. des Schuhüberziehers vorgesehen ist.

10. Bandage nach Anspruch 8 oder 9,
**dadurch gekennzeichnet,**
daß jedes Befestigungselement (11) als Öse, als Klettverschluß- oder Haftklebeorgan, als Druckknopf, als Knopfloch, als Band oder haken- oder knopfförmig ausgebildet ist.

11. Bandage nach einem der Ansprüche 8 bis 10,
**dadurch gekennzeichnet,**
daß der jeweilige Befestigungsabschnitt jedes Zugbandes (1) zur Verbindung mit dem Schuh (12) oder Schuhüberzieher ein Klettverschluß- (10, 10'), ein Haftklebe-, ein Knöpf-, ein Einhäng- oder ein Schnürverbindungselement umfaßt.

12. Bandage nach einem der Ansprüche 8 bis 11,
**dadurch gekennzeichnet,**
daß an jedem Zugband (1) eine Verbindungsschlaufe ausgebildet ist, die insbesondere über eine Klettverschlußverbindung (10, 10') verstellbar ist.

13. Bandage nach einem der vorhergehenden Ansprüche,
**dadurch gekennzeichnet,**
daß die Ringbandage (4) mit dem oder den Zugbändern (1) fest verbunden, insbesondere vernäht oder einstückig ausgebildet ist und/oder unter Ausbildung einer Y-Bandverzweigung oder einer X-Bandverzweigung verbunden ist.

14. Bandage nach einem der vorhergehenden Ansprüche,
**dadurch gekennzeichnet,**
daß die Ringbandage (4) mit dem oder den Zugbändern (1) über ein Ringelement (2) verbunden ist, mit welchem das jeweilige Zugband (1) fest verbunden, insbesondere vernäht ist oder durch welches das jeweilige Zugband (1) frei beweglich geschleift ist oder mit welchem das jeweilige Zugband (1) mittels einer insbesondere über eine Klettverschlußverbindung verstellbaren Verbindungsschlaufe verbunden ist.

15. Bandage nach einem der vorhergehenden Ansprüche,
**dadurch gekennzeichnet,**
daß die Ringbandage (4) aus zwei über Verschlußorgane, insbesondere Klettverschlußorgane (8, 8') verbundenen, am Zugband (1) fixierten Bändern besteht und/oder zumindest über einen Teilbereich ihres Umfangs gepolstert und/oder rückseitig verbreitert ausgeführt ist.

16. Bandage nach einem der vorhergehenden Ansprüche,
**dadurch gekennzeichnet,**
daß fußinnenseitig ein weiteres Zugband (1) vorgesehen ist, das mittels der Ringbandage (4) spannbar ist und dessen freies Ende einen weiteren Befestigungsabschnitt zur Befestigung an dem fußfesten Teil (12, 14) aufweist, und das sich insbesondere zumindest im wesentlichen in Richtung des Innenknöchels erstreckt.
